# EUROPEAN PATENT APPLICATION

(11) **EP 4 652 967 A1**
(43) Date of publication of application: **26.11.2025**
(21) Application number: 23917576.3
(22) Date of filing: 15.03.2023
(51) Int. Cl.: A61F 2/848

(54) **STENT**

(30) Priority: 19.01.2023 WO PCT/JP2023/001432
(71) Applicant: ASAHI INTECC CO., LTD., Seto-shi, Aichi, 489-0071 (JP)
(72) Inventor: MINAMI, Erina, Seto-shi, Aichi 489-0071 (JP); TOYOKAWA, Yoshihide, Seto-shi, Aichi 489-0071 (JP)
(74) Representative: Winter, Brandl - Partnerschaft mbB
(86) International application number: PCT/JP2023/010059
(87) International publication number: WO 2024/154363

(57) **Abstract**

A stent includes a tubular portion and a locking portion. The tubular portion is formed by weaving a wire, has a plurality of entwined portions at each of which the wire intersects with itself in a hook shape and is entwined with itself, and is configured to be expandable. The locking portion is configured to protrude to the outside of the tubular portion when the tubular portion is expanded. The locking portion has a first linear body that passes through a first hole formed in a first entwined portion that is one of the plurality of entwined portions.

## Description

### TECHNICAL FIELD

The technique disclosed in the present specification relates to a stent.

### BACKGROUND ART

For example, when a constricted part or an occluded part (hereinafter, collectively simply referred to as a "constricted part") occurs in a body lumen (a digestive organ such as a bile duct, a gallbladder, a pancreas, an esophagus, a duodenum, a small intestine, or a large intestine, a blood vessel, a ureter, a trachea, or the like), stent placement is used. The stent placement is a procedure for securing a lumen by placing a tubular stent at a position of the constricted part or a position bypassing the constricted part.

In general, a self-expanding stent is used for the stent placement. The self-expanding stent is a stent that contracts in the radial direction due to elasticity when a compressive force is applied, and expands in the radial direction when the compressive force is released. The self-expanding stent is formed by, for example, weaving a wire, and has a plurality of entwined portions at each of which the wire intersects with itself in a hook shape and is entwined with itself.

The self-expanding stent is mounted on a delivery system in a diameter-reduced state, is transported to a placement position, is self-expanded when being released from the delivery system, and is placed in that state. When the positional deviation (migration) of the stent occurs after the placement of the stent, the function of the stent is reduced or other problems are induced. Therefore, the stent is required to have a function of stably maintaining the placement position.

Conventionally, in order to suppress the positional deviation of the stent, a configuration in which locking portions having an anchor function are provided by intentionally not entwining a wire in a part of a plurality of entwined portion forming positions in the stent and bending the part from a tubular portion of the stent to the outer peripheral side has been proposed (for example, refer to Patent Literature 1).

### CITATION LIST

### Patent Literature

Patent Literature 1: WO 2020/194506 A1

### SUMMARY OF INVENTION

### TECHNICAL PROBLEM

The above-described conventional stent has a configuration in which the wire is not entwined with itself in a part of the plurality of entwined portion forming positions, and thus, the strength and durability may be decreased. In the above-described conventional stent, the anchor function of the locking portions may be decreased due to the movement of the locking portions.

The present specification discloses a technique capable of solving the above-described problems.

### SOLUTION TO PROBLEM

The technique disclosed in the present specification can be achieved as, for example, the following aspects.
(1) A stent disclosed in the present specification includes a tubular portion and a locking portion (first locking portion). The tubular portion is formed by weaving a wire, has a plurality of entwined portions at each of which the wire intersects with itself in a hook shape and is entwined with itself, and is configured to be expandable. The locking portion is configured to protrude to the outside of the tubular portion when the tubular portion is expanded. The locking portion has a first linear body that passes through a first hole formed in a first entwined portion that is one of the plurality of entwined portions.
   As described above, the stent includes the tubular portion formed by weaving the wire and the locking portion that protrudes to the outside of the tubular portion when the tubular portion is expanded. Thus, compared to a conventional configuration in which locking portions are provided by intentionally not entwining a wire in a part of a plurality of entwined portion forming positions, the positional deviation of the stent after placement can be suppressed by the anchor effect by the locking portion while suppressing a decrease in strength and durability of the tubular portion due to providing of the locking portion. In the stent, the locking portion has the first linear body that passes through the first hole formed in the first entwined portion. Thus, the movement of a portion of the first linear body passing through the first hole is restricted, thereby suppressing the movement of the linear body, and a decrease in the anchor function of the locking portion due to the movement can be suppressed.
(2) In the above-described stent, the first linear body may be constituted by a part of the wire. According to the present configuration, a joint member for joining the tubular portion and the first linear body is not necessary, and diameter of the stent in a diameter-reduced state can be reduced.
(3) In the above-described stent, the first linear body may be positioned in the same virtual plane. According to the present configuration, the stress concentration on a body wall by the first linear body can be alleviated, and the occurrence of bleeding or perforation in the body wall can be suppressed.
(4) In the above-described stent, the first linear body may pass through a second hole formed in a second entwined portion that is one of the plurality of entwined portions and arranged side by side with the first entwined portion in a circumferential direction of the tubular portion. According to the present configuration, the movement of a portion of the first linear body passing through the first hole and a portion of the first linear body passing through the second hole are restricted, thereby effectively suppressing the movement of the linear body, and a decrease in the anchor function of the locking portion due to the movement can be effectively suppressed.
(5) In the above-described stent, another entwined portion that is one of the plurality of entwined portions may be positioned between the first entwined portion and the second entwined portion in the circumferential direction of the tubular portion. According to the present configuration, the width of the first linear body can be made relatively wide, the stress concentration on the body wall by the first linear body can be alleviated, and the occurrence of bleeding or perforation in the body wall can be suppressed.
(6) In the above-described stent, the locking portion may have a second linear body that passes through the first hole and the second hole. According to the present configuration, the anchor effect by the locking portion can be increased, and the positional deviation of the stent after placement can be effectively suppressed.
(7) In the above-described stent, the second linear body may be constituted by a part of the wire. According to the present configuration, a joint member for joining the tubular portion and the second linear body is not necessary, and diameter of the stent in a diameter-reduced state can be reduced.
(8) In the above-described stent, a length of the first linear body and a length of the second linear body may be different from each other. According to the present configuration, a cover can be easily and reliably formed in an area surrounded by the linear bodies. Thus, for example, thickness of the cover formed in the area can be reduced.
(9) In the above-described stent, the locking portion may have a third linear body arranged side by side with the first linear body in the circumferential direction of the tubular portion and passing through a third hole formed in a third entwined portion that is one of the plurality of entwined portions, and, when viewed in a direction of a center axis of the tubular portion, the first linear body and the third linear body may be arranged so as to partially overlap each other. According to the present configuration, a portion of the locking portion in contact with the body wall has a planar spread, the stress concentration on the body wall by the locking portion can be effectively alleviated, and the occurrence of bleeding or perforation in the body wall can be effectively suppressed.
(10) The above-described stent may include a resin cover that covers at least a part of the tubular portion, and the cover may be arranged in an area surrounded by the first linear body constituting the locking portion. According to the present configuration, the stress concentration on the body wall by the first linear body can be further effectively alleviated, and the occurrence of bleeding or perforation in the body wall can be further effectively suppressed. The presence of the cover can prevent a tumor, granulation, or the like from entering the area surrounded by the first linear body, and thus the removal work of the stent can be facilitated.
(11) In the above-described stent, the first linear body may have a shape in which a width of a connection portion with the tubular portion is larger than a width of another portion. According to the present configuration, protruding of the first linear body to the outside when the stent is in a diameter-reduced state can be suppressed, and the accommodation property of the stent in a delivery system can be improved.
(12) The above-described stent may include a second locking portion that is arranged at a different position from a position of the locking portion in the direction of the center axis of the tubular portion and protrudes to the outside of the tubular portion when the tubular portion is expanded, the second locking portion may have a fourth linear body that passes through a fourth hole formed in a fourth entwined portion that is one of the plurality of entwined portions, and the fourth linear body may have a shape in which a width of a connection portion with the tubular portion is smaller than a width of another portion. According to the present configuration, a wide range portion of the linear body constituting the second locking portion comes into contact with the body wall, the stress concentration on the body wall by the linear body can be effectively alleviated, and the occurrence of bleeding or perforation in the body wall can be effectively suppressed. When the end portion of the stent is pulled in the case where the stent is occluded again, since the width of the connection portion of the linear body constituting the second locking portion is small, the linear body is easily reversed, and as a result, the removal of the stent can be facilitated.

The technique disclosed in the present specification can be achieved in various aspects, for example, in aspects of a stent, and a manufacturing method, a using method, or the like of a stent.

### BRIEF DESCRIPTION OF DRAWINGS

FIG. 1 is a perspective view illustrating an external appearance configuration of a stent 10 in a first embodiment.
FIG. 2 is an explanatory view illustrating a planar configuration of the stent 10 in the first embodiment.
FIG. 3 is a development view in which a main body part 100 in the stent 10 of the first embodiment is developed in a circumferential direction D1.
FIG. 4 is an explanatory view illustrating a weaving method of the main body part 100 in the first embodiment.
FIG. 5 is an explanatory view illustrating the weaving method of the main body part 100 in the first embodiment.
FIG. 6 is an explanatory view illustrating the weaving method of the main body part 100 in the first embodiment.
FIG. 7 is an explanatory view illustrating an example of a using method of the stent 10 in the first embodiment.
FIG. 8 is an explanatory view illustrating an external appearance configuration of a stent 10a in a second embodiment.
FIG. 9 is an explanatory view illustrating a planar configuration of the stent 10a in the second embodiment.
FIG. 10 is an explanatory view illustrating a weaving method of the main body part 100 of the stent 10a in the second embodiment.
FIG. 11 is an explanatory view illustrating an external appearance configuration of a stent 10b in a third embodiment.
FIG. 12 is an explanatory view illustrating an external appearance configuration of a stent 10c in a fourth embodiment.
FIG. 13 is an explanatory view illustrating an external appearance configuration of a stent 10d in a fifth embodiment.
FIG. 14 is an explanatory view illustrating a planar configuration of the stent 10d in the fifth embodiment.
FIG. 15 is an explanatory view illustrating an external appearance configuration of a stent 10e in a sixth embodiment.
FIG. 16 is a perspective view illustrating an external appearance configuration of a stent 10f in another embodiment.
FIG. 17 is an explanatory view illustrating a planar configuration of the stent 10f in the another embodiment.
FIG. 18 is a development view in which the main body part 100 in the stent 10f of the another embodiment is developed in the circumferential direction D1.
FIG. 19 is an explanatory view illustrating a weaving method of the main body part 100 in the stent 10f of the another embodiment.
FIG. 20 is an explanatory view illustrating the weaving method of the main body part 100 in the stent 10f of the another embodiment.

### EMBODIMENTS OF THE INVENTION

### A. First Embodiment:

### A-1. Configuration of Stent 10:

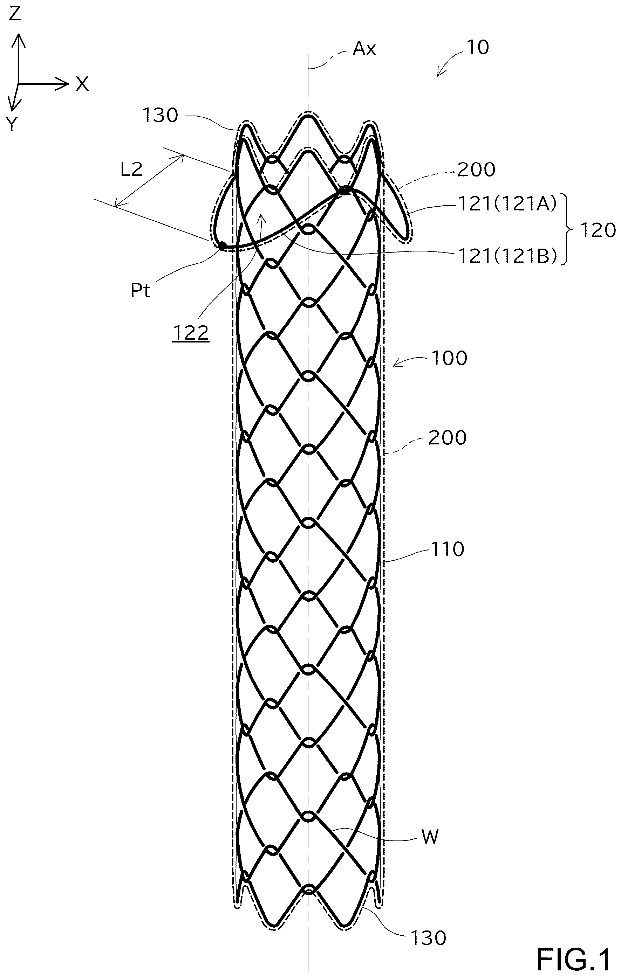

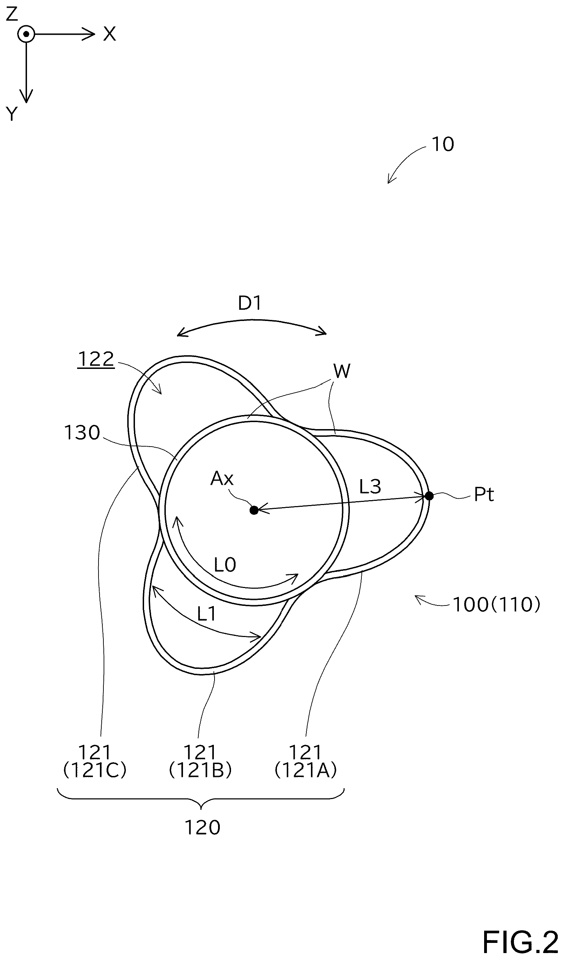

FIG. 1 is a perspective view illustrating an external appearance configuration of a stent 10 in a first embodiment, and FIG. 2 is an explanatory view illustrating a planar configuration of the stent 10 in the first embodiment. XYZ axes orthogonal to each other are illustrated in FIGS. 1 and 2. In the following description, for convenience, a Z-axis positive direction side may be referred to as an upper side, and a Z-axis negative direction side may be referred to as a lower side. FIG. 1 illustrates a state in which a center axis Ax of (a tubular portion 110 described below of) the stent 10 is parallel to the Z-axis, but the stent 10 is configured to be capable of being curved as a whole.

The stent 10 is a medical device that is placed at a position of a constricted part or a position bypassing the constricted part to secure a lumen, for example, when the constricted part occurs in a body lumen (a digestive organ such as a bile duct, a gallbladder, a pancreas, an esophagus, a duodenum, a small intestine, or a large intestine, a blood vessel, a ureter, a trachea, or the like). The stent 10 is a self-expanding stent that contracts in the radial direction due to elasticity when a compressive force is applied, and expands in the radial direction when the compressive force is released. The stent 10 in an expanded state is illustrated in FIGS. 1 and 2.

As illustrated in FIG. 1, the stent 10 of the present embodiment is a so-called covered stent, and includes a main body part 100 and a cover 200. For convenience, the cover 200 is shown by a dashed line in FIG. 1, and illustration of the cover 200 is omitted in FIG. 2. The cover 200 is formed of, for example, a resin such as polyurethane or silicone, and covers at least a part of the main body part 100. In the present embodiment, the cover 200 covers substantially the entire main body part 100.

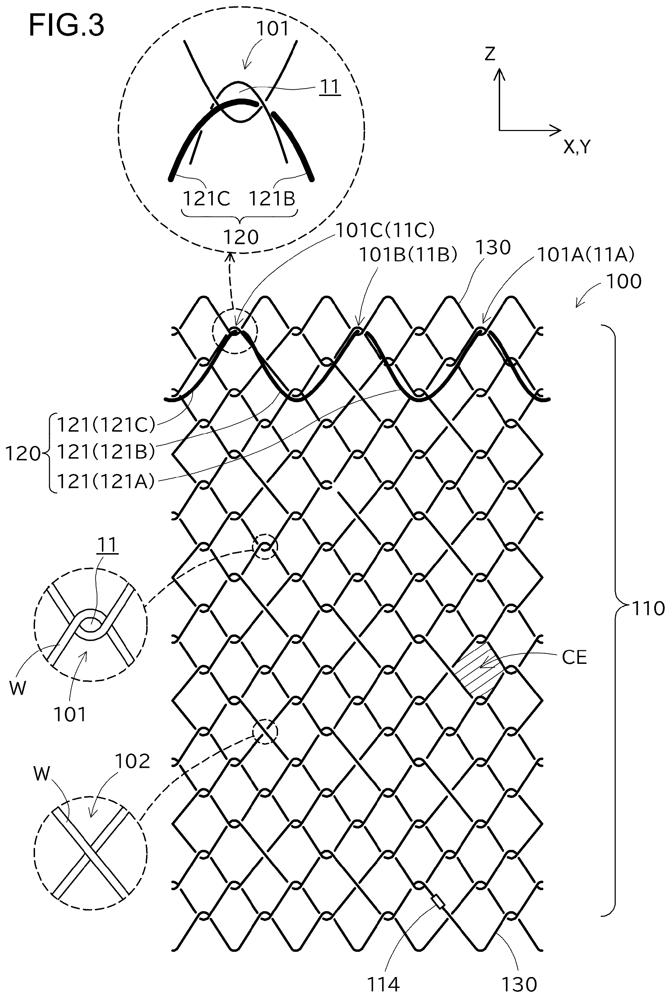

FIG. 3 is a development view in which the main body part 100 in the stent 10 of the first embodiment is developed in a circumferential direction D1. In the present specification, as illustrated in FIG. 2, the circumferential direction D1 means a circumferential direction around the center axis Ax of the tubular portion 110 of the main body part 100. The main body part 100 is a portion constituting a skeleton of the stent 10, and includes the tubular portion 110, a locking portion (first locking portion) 120, and a protruding portion 130.

The tubular portion 110 is a portion that expands in diameter to have a substantially cylindrical shape when the stent 10 is in the expanded state. In the expanded state of the stent 10, the radius of the tubular portion 110 is, for example, about 2 mm to 40 mm, and the length of the tubular portion 110 along the center axis Ax is, for example, about 20 mm to 200 mm. The radius of the tubular portion 110 is appropriately determined depending on the type, size, position, and the like of the body lumen in which the stent 10 is placed.

As illustrated in FIG. 3, the tubular portion 110 is formed by weaving a wire W. A weaving method of the tubular portion 110 is so-called hook weaving, and cells CE which are substantially rhombic holes surrounded by the wire W are regularly arranged. The tubular portion 110 has a plurality of intersecting portions 102 at each of which the wire W intersects without being entwined with itself, and a plurality of entwined portions 101 at each of which a portion of the wire W having a substantially V-shape convex upward and a portion of the wire W having a substantially V-shape convex downward intersect in a hook shape and are entwined with each other. In the entwined portion 101, a portion of the wire W convex upward and a portion of the wire W convex downward are connected to each other so as to be inseparable but relatively movable. Thus, the main body part 100 can be curved as a whole, can maintain the curved state, and can be easily placed in a curved living body lumen. In each entwined portion 101, a hole 11 penetrating in the radial direction of the tubular portion 110 is formed by the portion of the wire W convex upward and the portion of the wire W convex downward.

The protruding portion 130 is a substantially V-shaped portion formed of the wire W, and is a portion protruding upward from the upper end of the tubular portion 110 or a portion protruding downward from the lower end of the tubular portion 110. When the stent 10 is in the expanded state, the protruding portion 130 is substantially parallel to the center axis Ax of the tubular portion 110. In the present embodiment, six protruding portions 130 convex upward are arranged side by side continuously in the circumferential direction D1 at the upper end of the tubular portion 110, and six protruding portions 130 convex downward are arranged side by side continuously in the circumferential direction D1 at the lower end of the tubular portion 110.

The locking portion 120 is a portion that protrudes to the outside of the tubular portion 110 when the tubular portion 110 is expanded. The locking portion 120 has at least one linear body 121 formed of the wire W. In the present embodiment, the locking portion 120 has three linear bodies 121 (121A, 121B, and 121C). Each linear body 121 constituting the locking portion 120 may be referred to as a locking portion.

The three linear bodies 121 constituting the locking portion 120 are arranged side by side continuously in the circumferential direction D1 in the vicinity of the upper end of the tubular portion 110 (at a position slightly lower than the upper end). That is, one linear body 121 is arranged at each of three positions obtained by dividing the outer periphery of the tubular portion 110 into three. In the present embodiment, the three linear bodies 121 have substantially the same shape and size. Due to the presence of the linear bodies 121 of the locking portion 120, when the stent 10 is in the expanded state, the maximum radius of the main body part 100 (that is, a distance L3 from the center axis Ax of the tubular portion 110 to the outermost peripheral point Pt of each linear body 121 of the locking portion 120) is larger than the radius of the tubular portion 110. The distance L3 is, for example, equal to or more than 4 mm and equal to or less than 50 mm.

Each linear body 121 constituting the locking portion 120 is formed so as to pass through the hole 11 formed in the entwined portion 101. More specifically, one end portion (a connection portion with the tubular portion 110) of one linear body 121A passes through the hole 11 (hereinafter, referred to as a "hole 11A") formed in one entwined portion 101 (hereinafter, referred to as an "entwined portion 101A"), and the other end portion of the linear body 121A passes through the hole 11 (hereinafter, referred to as a "hole 11B") formed in another entwined portion 101 (hereinafter, referred to as an "entwined portion 101B") secondly positioned along the circumferential direction D1 when viewed from the entwined portion 101A. That is, both end portions of the linear body 121A pass through the two holes 11A and 11B formed in the two adjacent entwined portions 101A and 101B with another entwined portion 101 interposed therebetween in the circumferential direction D1.

Similarly, one end portion of another linear body 121B passes through the hole 11B formed in the above-described entwined portion 101B, and the other end portion of the linear body 121B passes through the hole 11 (hereinafter, referred to as a "hole 11C") formed in another entwined portion 101 (hereinafter, referred to as an "entwined portion 101C") secondly positioned along the circumferential direction D1 when viewed from the entwined portion 101B. One end portion of still another linear body 121C passes through the hole 11C formed in the above-described entwined portion 101C, and the other end portion of the linear body 121C passes through the hole 11A formed in the above-described entwined portion 101A that is the entwined portion 101 secondly positioned along the circumferential direction D1 when viewed from the entwined portion 101C.

As described above, in the present embodiment, both ends of each of the three linear bodies 121 constituting the locking portion 120 pass through the holes 11 formed in the entwined portions 101. The linear body 121 passing through the hole 11 formed in the entwined portion 101 means that a part of the linear body 121 reaches the hollow portion of the hole 11, and is not necessarily limited to a form in which a part of the linear body 121 passes through the hole 11. One linear body 121 is an example of a first linear body, the hole 11 of one entwined portion 101, through which one end portion of the one linear body passes, is an example of a first hole of a first entwined portion, and the hole 11 of another entwined portion 101, through which the other end portion of the one linear body passes, is an example of a second hole of a second entwined portion.

Each linear body 121 constituting the locking portion 120 is configured to be positioned in the same virtual plane. The linear body 121 being positioned in the same virtual plane is not limited to a state in which the linear body 121 is positioned strictly in the same virtual plane, but includes a state in which the linear body 121 is positioned substantially in the same virtual plane, in particular, a state in which a distance from a freely-selected point on the linear body 121 to one virtual plane is equal to or less than 3 mm.

As illustrated in FIG. 1, when the stent 10 is in the expanded state, each linear body 121 constituting the locking portion 120 is configured to extend obliquely downward from the connection portion with the tubular portion 110. The inclination of each linear body 121 when the stent 10 is in the expanded state (that is, the angle formed by the center axis Ax of the tubular portion 110 and the above-described virtual plane) is, for example, equal to or more than 20 degrees and equal to or less than 80 degrees, and more specifically, for example, equal to or more than 30 degrees and equal to or less than 70 degrees. By adopting a configuration in which each linear body 121 extends obliquely downward from the connection portion with the tubular portion 110, when the stent 10 is reduced in diameter and housed in a housing tool (sheath) constituting a delivery system, the amount of deformation of each linear body 121 can be reduced, and the load on the inner wall of the housing tool by each linear body 121 can be reduced.

As illustrated in FIG. 2, when viewed in the direction of the center axis Ax of the tubular portion 110, the entire shape of each linear body 121 constituting the locking portion 120 is a gentle bowl shape. That is, the entire shape of each linear body 121 is a shape in which the width becomes narrower as a distance from the connection portion with the tubular portion 110 is increased in the radial direction. Thus, a width L0 of the connection portion with the tubular portion 110 in each linear body 121 is larger than a width L1 of another portion. In the present embodiment, the width L0 of the connection portion with the tubular portion 110 in each linear body 121 is larger than a protruding length L2 of each linear body 121. However, the present embodiment is not limited thereto. In the present specification, the width of the linear body 121 means a width along the circumferential direction D1 of the shape (for example, a bowl shape) defined by the entire linear body 121, and the protruding length of the linear body 121 means a protruding length along the above-described virtual plane. In the present embodiment, each linear body 121 is bent in a curved shape over the entire length, and does not have a folded portion. As described above, since both end portions of each linear body 121 pass through two holes 11 formed in two adjacent entwined portions 101 with another entwined portion 101 interposed therebetween instead of two entwined portions 101 directly adjacent to each other in the circumferential direction D1, each linear body 121 has a mountain shape having a relatively wide bottom width. The cover 200 is arranged in the entire area 122 surrounded by each linear body 121.

In the present embodiment, the tubular portion 110, the locking portion 120, and the protruding portion 130 constituting the main body part 100 are continuously formed by weaving one wire W. However, these may be formed by separate wires W. The wire W is formed of a superelastic alloy such as a nickel-titanium (Ni-Ti) alloy. The wire W may be formed of another metal such as stainless steel, tantalum, titanium, a cobalt-chromium alloy, or a magnesium alloy, or may be formed of a resin such as polyolefin, polyester, or a fluororesin. The diameter of the wire W is, for example, about 0.05 mm to 0.5 mm.

### A-2. Manufacturing Method of Stent 10:

Next, an example of a manufacturing method of the stent 10 of the present embodiment will be described. First, the main body part 100 is manufactured by hook-weaving the wire W. A manufacturing method of a stent by hook weaving is known as described in, for example, Japanese Patent No. 3708923, and will be briefly described below.

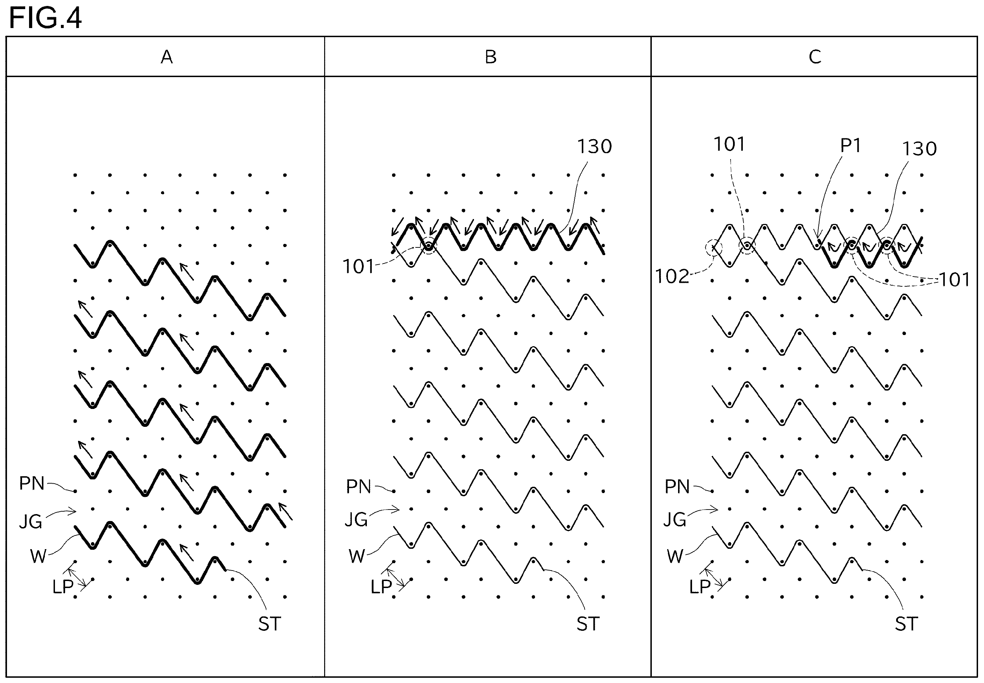

FIGS. 4 to 6 are explanatory views illustrating a weaving method of the main body part 100 in the first embodiment. In each column of FIGS. 4 to 6, a procedure of manufacturing the main body part 100 by weaving the wire W using a jig JG in which a plurality of pins PN are erected on the outer peripheral surface of a cylindrical member is illustrated in the form of a development view. Hereinafter, a distance between two pins PN adjacent to each other in an oblique direction in the jig JG is referred to as a diagonal distance LP.

First, as illustrated in the column A of FIG. 4, an end portion of the wire W is fixed to the pin PN at a predetermined position of the jig JG as a start point ST, and the wire W is extended and hooked on the pins PN in a zigzag manner from the start point ST. More specifically, the wire W is extended obliquely upward and leftward from the start point ST to be hooked on the upper side of the pin PN, and then, an operation of extending the wire W obliquely downward and leftward by 1 × LP to be hooked on the lower side of the pin PN and an operation of extending the wire W obliquely upward and leftward by 2 × LP to be hooked on the upper side of the pin PN are repeated until the wire W reaches a position corresponding to the upper end of the tubular portion 110.

Next, as illustrated in the column B of FIG. 4, following the formation state of the wire W in the column A of FIG. 4, the wire W is extended and hooked on the pins PN in a zigzag manner from the position corresponding to the upper end of the tubular portion 110 so that six V-shaped portions (the protruding portions 130) convex upward are formed. More specifically, the six protruding portions 130 are formed by an operation of extending the wire W obliquely upward and leftward by 1 × LP to be hooked on the upper side of the pin PN and an operation of extending the wire W obliquely downward and leftward by 1 × LP to be hooked on the lower side of the pin PN. At this time, the wire W intersects in a hook shape at a position of the pin PN around which the wire W has already been wound, thereby forming the entwined portion 101. As for this point, the same applies hereinafter. The wire W intersects without being entwined with itself at a position where the wire W passes obliquely without being wound around the pin PN, thereby forming the intersecting portion 102. Also, as for this point, the same applies hereinafter.

Next, as illustrated in the column C of FIG. 4, following the formation state of the wire W in the column B of FIG. 4, the wire W is extended and hooked on the pins PN in a zigzag manner so that three V-shaped portions convex downward are formed. More specifically, an operation of extending the wire W obliquely downward and leftward by 1 × LP to be hooked on the lower side of the pin PN and an operation of extending the wire W obliquely upward and leftward by 1 × LP to be hooked on the upper side of the pin PN are repeated three times. The completion position of the operations is a locking portion formation start point P1. At this point, three entwined portions 101 (the holes 11) are formed.

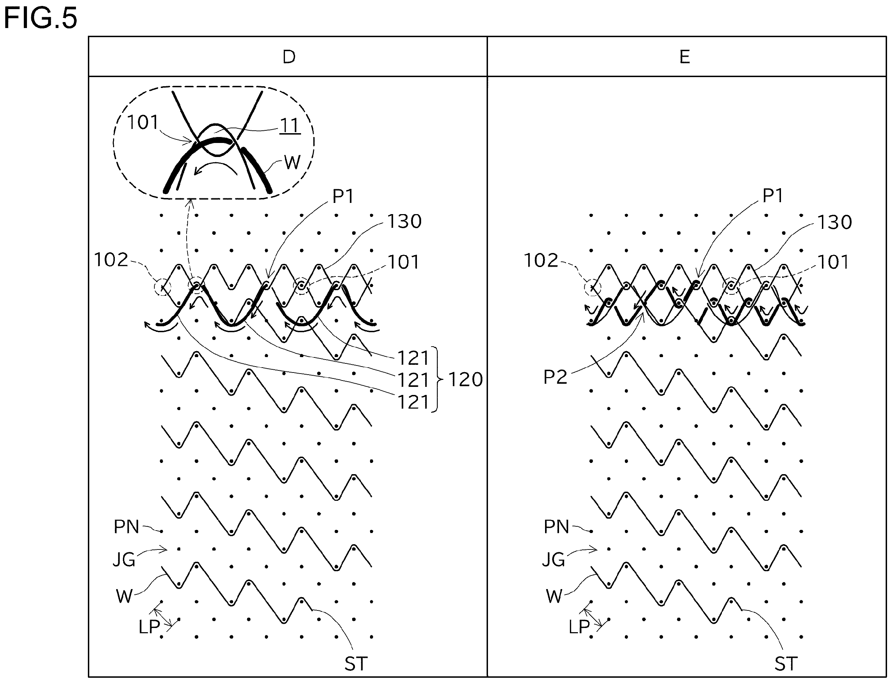

Next, as illustrated in the column D of FIG. 5, following the formation state of the wire W in the column C of FIG. 4, the wire W is extended and hooked on the pins PN in a zigzag manner so that three large V-shaped portions (the linear bodies 121 of the locking portion 120) convex downward are formed. More specifically, an operation of extending the wire W obliquely downward and leftward by 2 × LP to be hooked on the lower side of the pin PN and an operation of extending the wire W obliquely upward and leftward by 2 × LP to be hooked on the upper side of the pin PN are repeated three times. At this time, the wire W is passed through the hole 11 at a position where the hole 11 of the entwined portion 101 is formed. At the time of completion of the operations, the wire W has taken one lap and returns to the locking portion formation start point P1. At this position, the hole 11 of the entwined portion 101 is formed afterward together with the wire W arranged by the operations illustrated in the column B of FIG. 4, so that the wire W constituting the end portion of the linear body 121 passes through the hole 11 even at this position. Accordingly, the both end portions of each of the three linear bodies 121 pass through the holes 11.

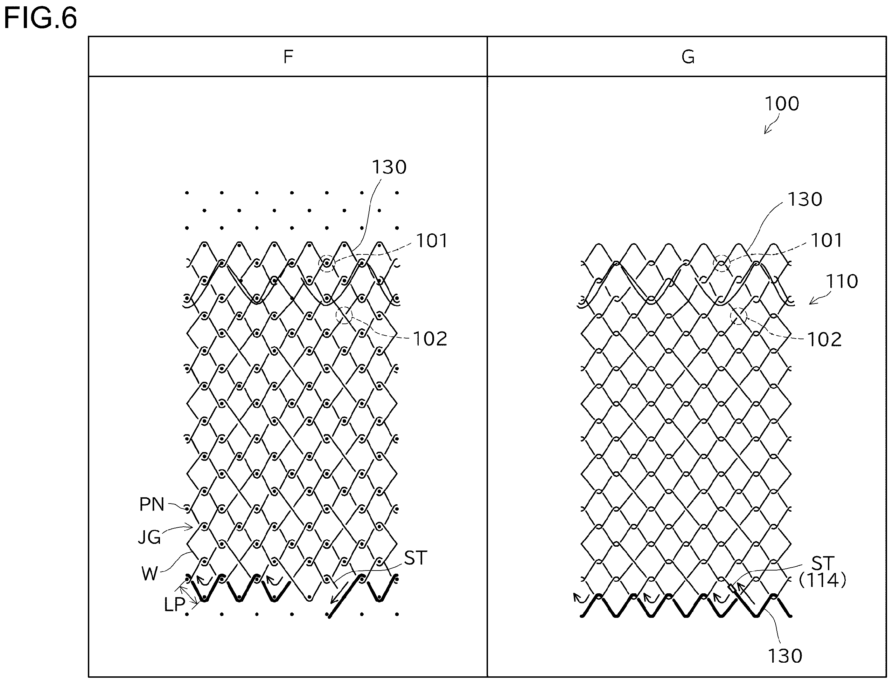

Next, as illustrated in the column E of FIG. 5 and the column F of FIG. 6, weaving of the tubular portion 110 is performed. In particular, following the formation state of the wire W in the column D of FIG. 5, an operation of extending the wire W obliquely downward and leftward by 1 × LP to be hooked on the lower side of the pin PN, an operation of extending the wire W obliquely upward and leftward by 1 × LP to be hooked on the upper side of the pin PN, and an operation of extending the wire W obliquely downward and leftward by 1 × LP to be hooked on the lower side of the pin PN are performed, so that the tip of the wire W is positioned at a reference point P2. Thereafter, an operation of extending the wire W obliquely downward and leftward by 1 × LP to be hooked on the lower side of the pin PN and an operation of extending the wire W obliquely upward and leftward by 1 × LP to be hooked on the upper side of the pin PN are repeated four times, and then, the wire W is extended obliquely downward and leftward by 2 × LP. Such operations are repeated until the wire W reaches a position corresponding to the lower end of the tubular portion 110. At this time, in an area in which each linear body 121 has already been formed, the wire W is woven so as to pass through the back side of each linear body 121.

Next, as illustrated in the column F of FIG. 6, the wire W is extended and hooked on the pins PN in a zigzag manner so that six V-shaped portions (the protruding portions 130) convex downward are formed. That is, the protruding portions 130 are formed by an operation of extending the wire W obliquely downward and leftward by 1 × LP to be hooked on the lower side of the pin PN and an operation of extending the wire W obliquely upward and leftward by 1 × LP to be hooked on the upper side of the pin PN. After the sixth protruding portion 130 is formed, the wire W is extended to the start point ST, and both ends of the wire W are connected at the start point ST by, for example, forming a caulking portion 114 by caulking. By the above-described steps, the main body part 100 before processing of the locking portion 120 is manufactured.

Next, for example, by using a disc-shaped mold, the linear bodies 121 of the locking portion 120 before processing are fixed in a state of being lifted so as to protrude from the tubular portion 110 to the outer peripheral side, and heat treatment for shape memory (for example, at 350 to 600°C for 5 to 30 minutes) is performed. Accordingly, the linear bodies 121 of the locking portion 120 are formed so as to protrude from the tubular portion 110 to the outer peripheral side during expansion.

Finally, the cover 200 is formed at a predetermined position of the main body part 100. By the above-described steps, the manufacturing of the stent 10 of the present embodiment is completed.

### A-3. Using Method of Stent 10:

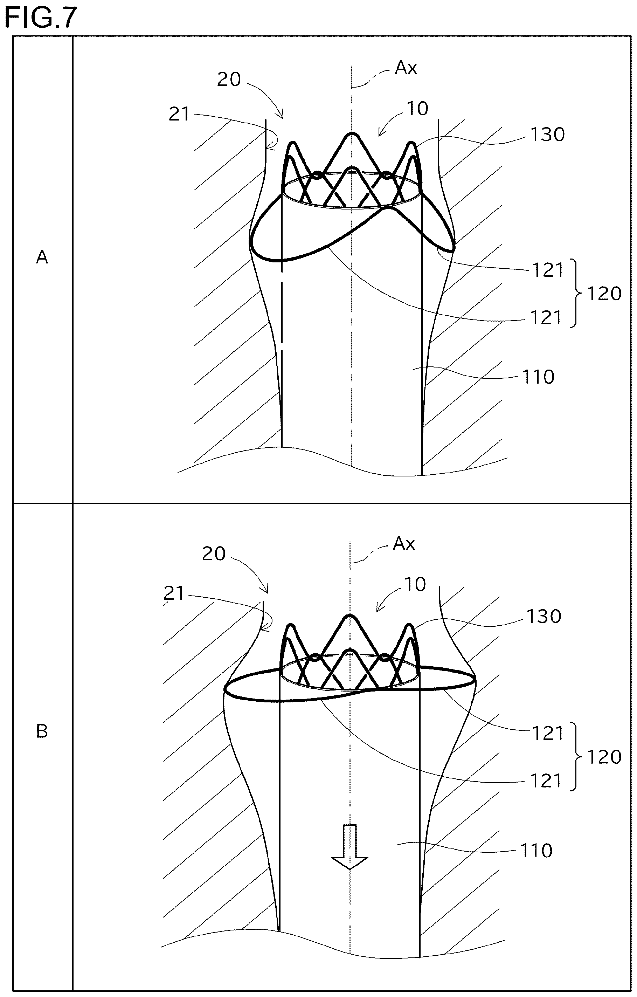

Next, an example of a using method of the stent 10 will be described. FIG. 7 is an explanatory view illustrating an example of a using method of the stent 10 in the first embodiment. In FIG. 7, illustration of a part of the wire W is omitted.

First, the stent 10 is mounted on a delivery system in a diameter-reduced state. In the diameter-reduced state of the stent 10, the linear bodies 121 of the locking portion 120 are folded so as not to protrude to the outside of the tubular portion 110. Next, the stent 10 is transported by the delivery system to a placement position (a position of a constricted part or a position bypassing the constricted part) in a living body lumen 20 illustrated in FIG. 7, and the stent 10 is released from the delivery system at the placement position. Accordingly, the stent 10 is in an expanded state due to self-expandability. When the stent 10 is in the expanded state, the linear bodies 121 of the locking portion 120 protrude to the outside of the tubular portion 110. Thus, as illustrated in the column A of FIG. 7, the linear bodies 121 of the locking portion 120 come into contact with a body wall 21 of the living body lumen 20 and functions as an anchor, and the positional deviation (migration) of the stent 10 is suppressed. As described above, since each linear body 121 of the locking portion 120 has a bowl shape, a wide range portion of each linear body 121 comes into contact with the body wall 21, the stress concentration on the body wall 21 is alleviated, and the occurrence of bleeding or perforation in the body wall 21 can be suppressed.

Each linear body 121 of the locking portion 120 is in a posture extending obliquely downward from the connection portion with the tubular portion 110. Thus, as illustrated in the column B of FIG. 7, when a downward force acts on the stent 10, the angle of each linear body 121 changes with the connection portion with the tubular portion 110 as a fulcrum, and the inclination with respect to the tubular portion 110 increases. As a result, the outer diameter of the stent 10 is further increased, the anchor function by the linear bodies 121 is increased, and the positional deviation of the stent 10 is further effectively suppressed.

### A-4. Effects of First Embodiment:

As described above, the stent 10 of the present embodiment includes the tubular portion 110 and the locking portion 120. The tubular portion 110 is formed by weaving the wire W. The tubular portion 110 has the plurality of entwined portions 101 at each of which the wire W intersects with itself in a hook shape and is entwined with itself, and is configured to be expandable. The locking portion 120 is configured to protrude to the outside of the tubular portion 110 when the tubular portion 110 is expanded. The locking portion 120 has the linear body (hereinafter also referred to as a "first linear body") 121 that passes through the hole (hereinafter also referred to as a "first hole") 11 formed in one entwined portion (hereinafter also referred to as a "first entwined portion") 101 among the plurality of entwined portions 101.

As described above, the stent 10 of the present embodiment includes the tubular portion 110 formed by weaving the wire W and the locking portion 120 that protrudes to the outside of the tubular portion 110 when the tubular portion 110 is expanded. Thus, compared to a conventional configuration in which locking portions are provided by intentionally not entwining a wire W in a part of a plurality of entwined portion forming positions, the positional deviation of the stent 10 after placement can be suppressed by the anchor effect by the locking portion 120 while suppressing a decrease in strength and durability of the tubular portion 110 due to providing of the locking portion 120. The locking portion 120 has the first linear body 121 that passes through the first hole 11 formed in the first entwined portion 101. Thus, the movement of a portion of the first linear body 121 passing through the first hole 11 is restricted, thereby suppressing the movement of the linear body 121, and a decrease in the anchor function of the locking portion 120 due to the movement can be suppressed.

In the stent 10 of the present embodiment, the first linear body 121 of the locking portion 120 is constituted by a part of the wire W for forming the tubular portion 110. Thus, according to the stent 10 of the present embodiment, a joint member for joining the tubular portion 110 and the first linear body 121 is not necessary, and diameter of the stent 10 in a diameter-reduced state can be reduced.

In the stent 10 of the present embodiment, the first linear body 121 of the locking portion 120 is configured to be positioned in the same virtual plane. Thus, according to the stent 10 of the present embodiment, the stress concentration on the body wall by the first linear body 121 can be alleviated, and the occurrence of bleeding or perforation in the body wall can be suppressed.

In the stent 10 of the present embodiment, the first linear body 121 of the locking portion 120 passes through the hole (hereinafter also referred to as a "second hole") 11 formed in another entwined portion (hereinafter also referred to as a "second entwined portion") 101 that is one of the plurality of entwined portions 101 and arranged side by side with the first entwined portion 101 in the circumferential direction D1 of the tubular portion 110. Thus, according to the stent 10 of the present embodiment, the movement of a portion of the first linear body 121 passing through the first hole 11 and a portion of the first linear body 121 passing through the second hole 11 are restricted, thereby effectively suppressing the movement of the linear body 121, and a decrease in the anchor function of the locking portion 120 due to the movement can be effectively suppressed.

In the stent 10 of the present embodiment, another entwined portion 101 is positioned between the first entwined portion 101 and the second entwined portion 101 in the circumferential direction D1 of the tubular portion 110. Thus, according to the stent 10 of the present embodiment, the width of the first linear body 121 can be made relatively wide, the stress concentration on the body wall by the first linear body 121 can be alleviated, and the occurrence of bleeding or perforation in the body wall can be suppressed.

The stent 10 of the present embodiment further includes the resin cover 200 that covers at least a part of the tubular portion 110. The cover 200 is also arranged in the area 122 surrounded by the first linear body 121 constituting the locking portion 120. Thus, according to the stent 10 of the present embodiment, the stress concentration on the body wall by the first linear body 121 can be further effectively alleviated, and the occurrence of bleeding or perforation in the body wall can be further effectively suppressed. The presence of the cover 200 can prevent a tumor, granulation, or the like from entering the area 122 surrounded by the first linear body 121, and thus the facilitation of the removal work of the stent 10 can be achieved.

In the stent 10 of the present embodiment, the first linear body 121 has a shape in which the width of the connection portion with the tubular portion 110 is larger than the width of another portion. Thus, according to the stent 10 of the present embodiment, protruding of the first linear body 121 to the outside when the diameter of the stent 10 is reduced can be suppressed, and the accommodation property of the stent 10 in the delivery system can be improved.

### B. Second Embodiment:

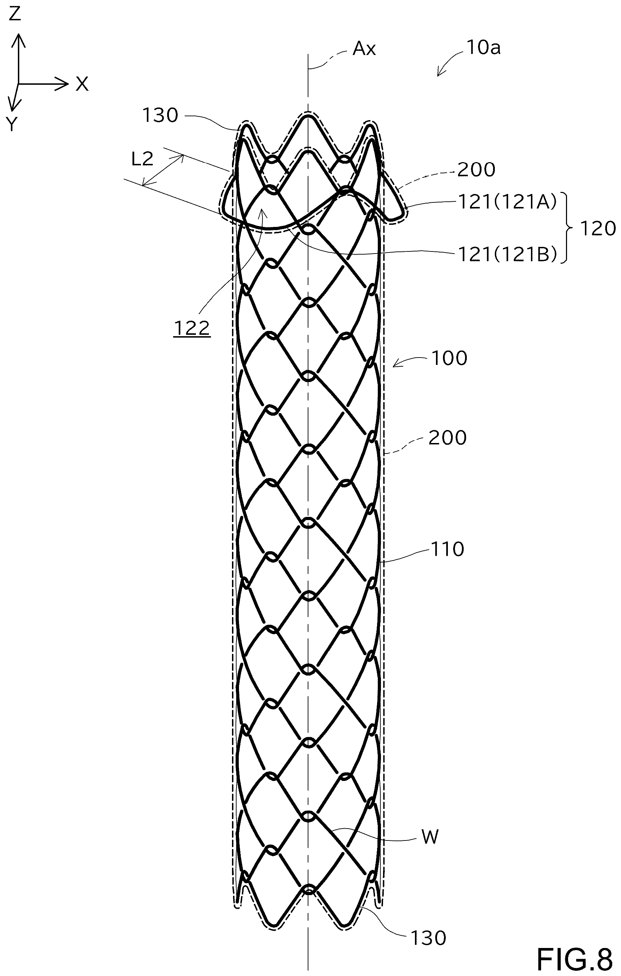

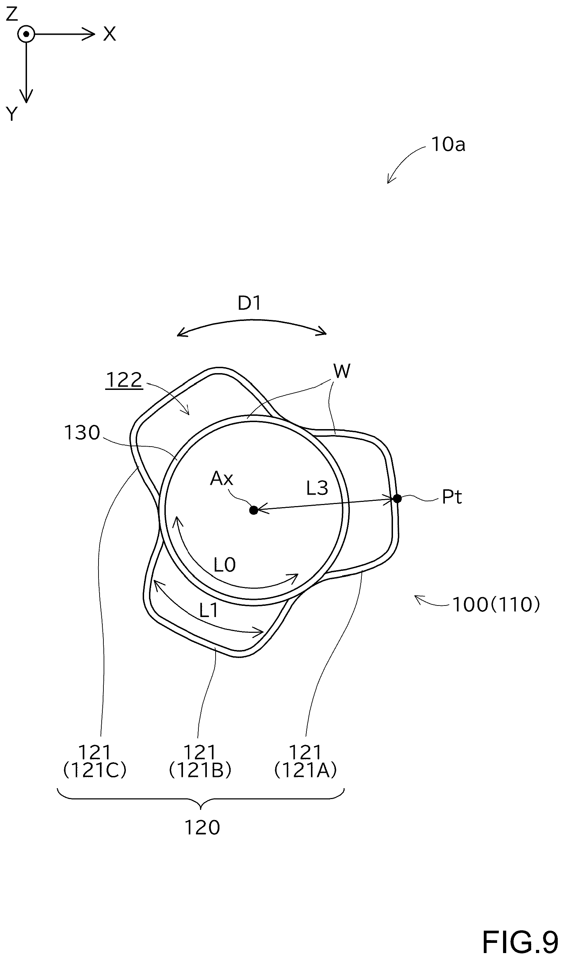

FIG. 8 is an explanatory view illustrating an external appearance configuration of a stent 10a in a second embodiment, and FIG. 9 is an explanatory view illustrating a planar configuration of the stent 10a in the second embodiment. Hereinafter, in the configuration of the stent 10a of the second embodiment, the same components as those of the stent 10 of the first embodiment described above will be denoted by the same reference signs, and the description thereof will be omitted as appropriate.

The stent 10a of the second embodiment is different from the stent 10 of the first embodiment in the shape of each linear body 121 of the locking portion 120. In particular, in the stent 10a of the second embodiment, when viewed in the direction of the center axis Ax of the tubular portion 110, the entire shape of each linear body 121 constituting the locking portion 120 is a gentle substantially trapezoidal shape. That is, the outermost peripheral portion of each linear body 121 has a substantially flat shape. Also in the stent 10a of the second embodiment, similarly to the stent 10 of the first embodiment, a width L0 of the connection portion with the tubular portion 110 in each linear body 121 is larger than a width L1 of another portion. The width L0 of the connection portion with the tubular portion 110 in each linear body 121 is larger than a protruding length L2 of each linear body 121. However, the present embodiment is not limited thereto.

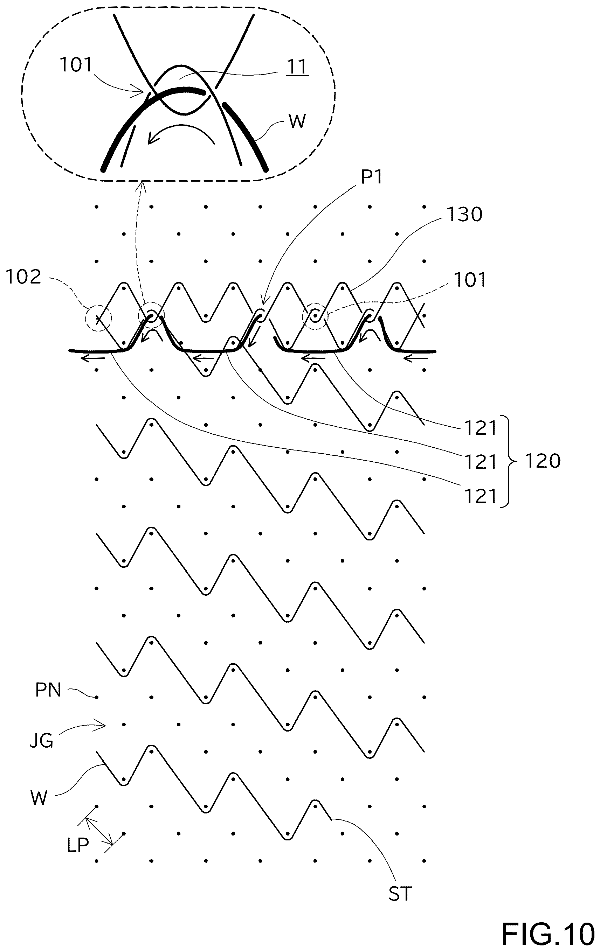

FIG. 10 is an explanatory view illustrating a weaving method of the main body part 100 of the stent 10a in the second embodiment. The weaving method of the main body part 100 in the second embodiment is different from the weaving method of the main body part 100 in the first embodiment in the forming step of the linear bodies 121 of the locking portion 120 (the step illustrated in the column D of FIG. 5 in the first embodiment). In particular, as illustrated in FIG. 10, in the forming step of the linear bodies 121 of the locking portion 120, the wire W is extended and hooked on the pins PN in a zigzag manner so that three large trapezoidal portions (the linear bodies 121 of the locking portion 120) convex downward are formed. More specifically, an operation of extending the wire W obliquely downward and leftward by 1 × LP to be hooked on the lower side of the pin PN, an operation of extending the wire leftward to be hooked on the adjacent pin PN, and an operation of extending the wire W obliquely upward and leftward by 1 × LP to be hooked on the upper side of the pin PN are repeated three times. Other steps are the same as those of the first embodiment. By such a manufacturing method, the stent 10a having the configuration illustrated in FIGS. 8 and 9 can be manufactured.

In the stent 10a of the second embodiment, similarly to the stent 10 of the first embodiment, the locking portion 120 has the first linear body 121 that passes through the first hole 11 formed in the first entwined portion 101 that is one of the plurality of entwined portions 101. Thus, the movement of a portion of the first linear body 121 passing through the first hole 11 is restricted, thereby suppressing the movement of the linear body 121, and a decrease in the anchor function of the locking portion 120 due to the movement can be suppressed.

### C. Third Embodiment:

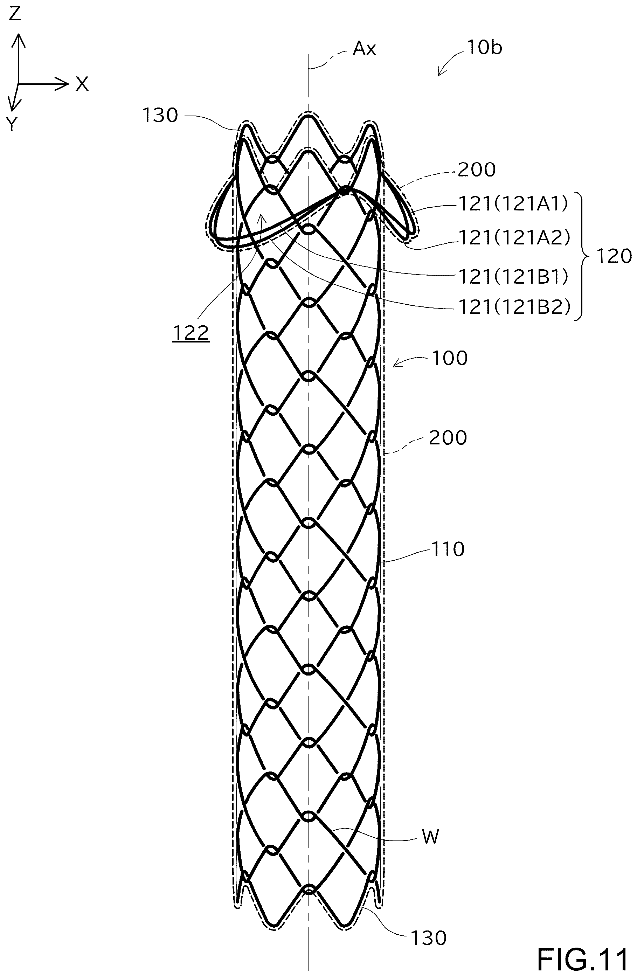

FIG. 11 is an explanatory view illustrating an external appearance configuration of a stent 10b in a third embodiment. Hereinafter, in the configuration of the stent 10b of the third embodiment, the same components as those of the stent 10 of the first embodiment described above will be denoted by the same reference signs, and the description thereof will be omitted as appropriate.

The stent 10b of the third embodiment is different from the stent 10 of the first embodiment in the configuration of the locking portion 120. In particular, in the stent 10b of the third embodiment, the linear body 121 is doubly formed at each position where the linear body 121 of the locking portion 120 is formed. That is, the locking portion 120 has six linear bodies 121, the six linear bodies 121 constitute three pairs, and each of the three pairs of linear bodies 121 is arranged at each of three positions obtained by dividing the outer periphery of the tubular portion 110 into three. For example, two linear bodies 121A1 and 121A2 are doubly formed at a certain position on the outer periphery of the tubular portion 110, and two linear bodies 121B1 and 121B2 are doubly formed at another position. One of the pair of linear bodies 121 arranged at each position is an example of a first linear body, and the other is an example of a second linear body. The linear bodies 121 arranged at each position have substantially the same length. In the present embodiment, the two linear bodies 121 connected to the same position around the tubular portion 110 are positioned in the same virtual plane. Each linear body 121 of the locking portion 120 is constituted by a part of the wire W for forming the tubular portion 110.

The stent 10b having such a configuration can be manufactured by performing the operation of extending the wire W to be hooked on the pins PN in a zigzag manner so that three large V-shaped portions convex downward are formed by two laps, in the forming step of the linear bodies 121 of the locking portion 120 in the manufacturing method of the stent 10 of the first embodiment described above (the step illustrated in the column D of FIG. 5).

In the stent 10b of the third embodiment, similarly to the stent 10 of the first embodiment, the locking portion 120 has the first linear body 121 that passes through the first hole 11 formed in the first entwined portion 101 that is one of the plurality of entwined portions 101. Thus, the movement of a portion of the first linear body 121 passing through the first hole 11 is restricted, thereby suppressing the movement of the linear body 121, and a decrease in the anchor function of the locking portion 120 due to the movement can be suppressed.

In the stent 10b of the third embodiment, in addition to the first linear body 121 that passes through the first hole 11 formed in the first entwined portion 101 and the second hole 11 formed in the second entwined portion 101, the locking portion 120 has another linear body (hereinafter, also referred to as a "second linear body") 121 that passes through the first hole 11 and the second hole 11. Thus, according to the stent 10b of the third embodiment, the anchor effect by the locking portion 120 can be increased, and the positional deviation of the stent 10b after placement can be effectively suppressed.

### D. Fourth Embodiment:

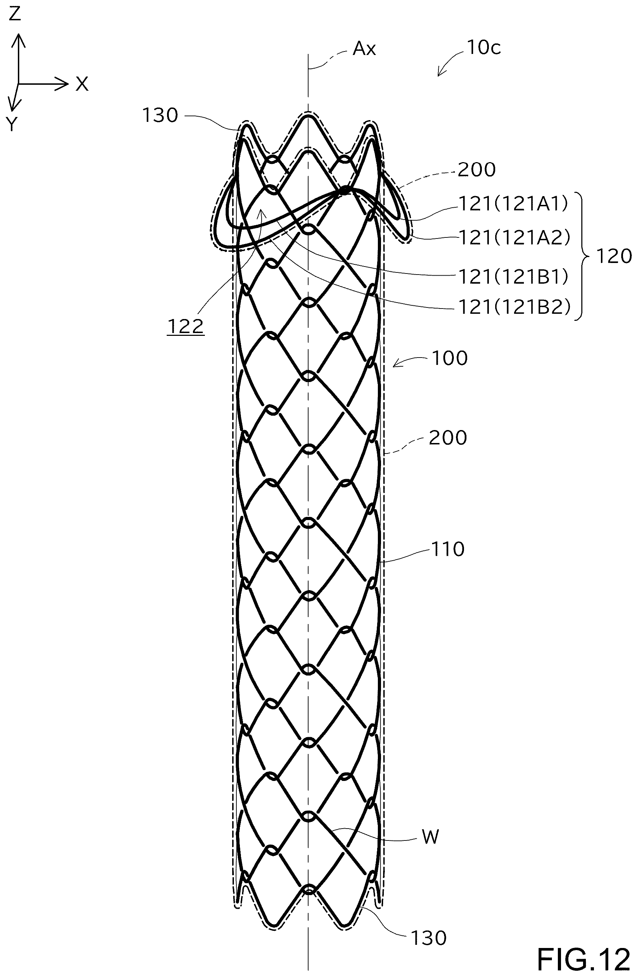

FIG. 12 is an explanatory view illustrating an external appearance configuration of a stent 10c in a fourth embodiment. Hereinafter, in the configuration of the stent 10c of the fourth embodiment, the same components as those of the stent 10b of the third embodiment described above will be denoted by the same reference signs, and the description thereof will be omitted as appropriate.

The stent 10c of the fourth embodiment is different from the stent 10b of the third embodiment in the configuration of the locking portion 120. In particular, in the stent 10c of the fourth embodiment, similarly to the third embodiment, the linear body 121 is doubly formed at each position where the linear body 121 of the locking portion 120 is formed. However, in the stent 10c of the fourth embodiment, the pair of linear bodies 121 (for example, the linear bodies 121A1 and 121A2) arranged at each position have different lengths. That is, at each position around the tubular portion 110, in the area 122 surrounded by one linear body 121, the other linear body 121 is arranged. In the present embodiment, the two linear bodies 121 connected to the same position around the tubular portion 110 are positioned in the same virtual plane.

The stent 10c having such a configuration can be manufactured by performing the operation of extending the wire W to be hooked on the pins PN in a zigzag manner so that three large V-shaped portions convex downward are formed by two laps while changing the size of the V-shaped portions for each lap, in the forming step of the linear bodies 121 of the locking portion 120 in the manufacturing method of the stent 10b of the third embodiment described above (the step illustrated in the column D of FIG. 5).

In the stent 10c of the fourth embodiment, similarly to the stent 10b of the third embodiment, the locking portion 120 has the first linear body 121 that passes through the first hole 11 formed in the first entwined portion 101 that is one of the plurality of entwined portions 101. Thus, the movement of a portion of the first linear body 121 passing through the first hole 11 is restricted, thereby suppressing the movement of the linear body 121, and a decrease in the anchor function of the locking portion 120 due to the movement can be suppressed.

In the stent 10c of the fourth embodiment, similarly to the stent 10b of the third embodiment, in addition to the first linear body 121 that passes through the first hole 11 formed in the first entwined portion 101 and the second hole 11 formed in the second entwined portion 101, the locking portion 120 has the second linear body 121 that passes through the first hole 11 and the second hole 11. Thus, the anchor effect by the locking portion 120 can be increased, and the positional deviation of the stent 10c after placement can be effectively suppressed.

In the stent 10c of the fourth embodiment, since the plurality of linear bodies 121 connected to the same position on the tubular portion 110 have different lengths, the cover 200 can be easily and reliably formed in the area 122 surrounded by the linear bodies 121. Thus, for example, thickness of the cover 200 formed in the area 122 can be reduced.

### E. Fifth Embodiment:

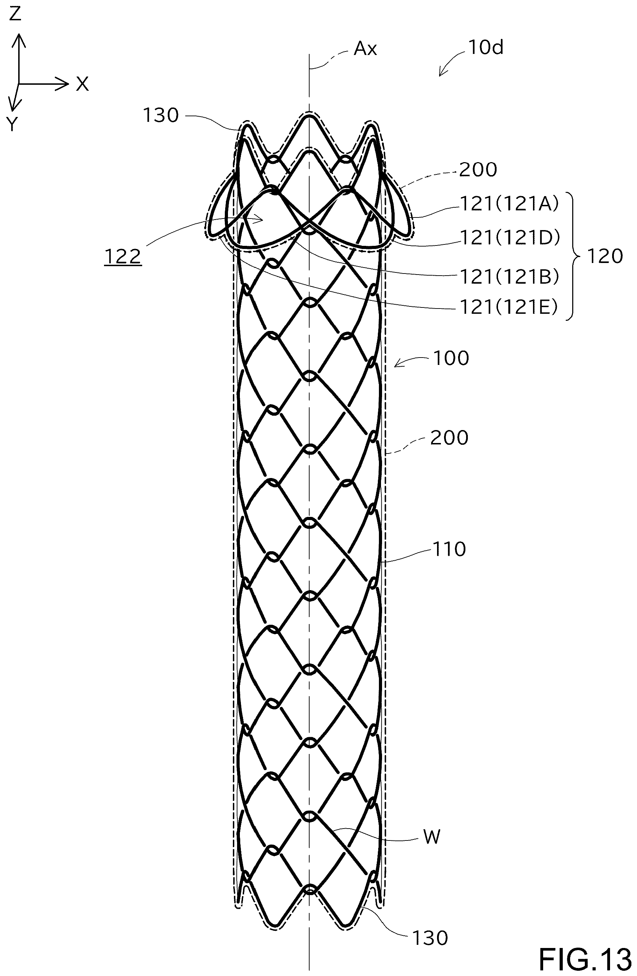

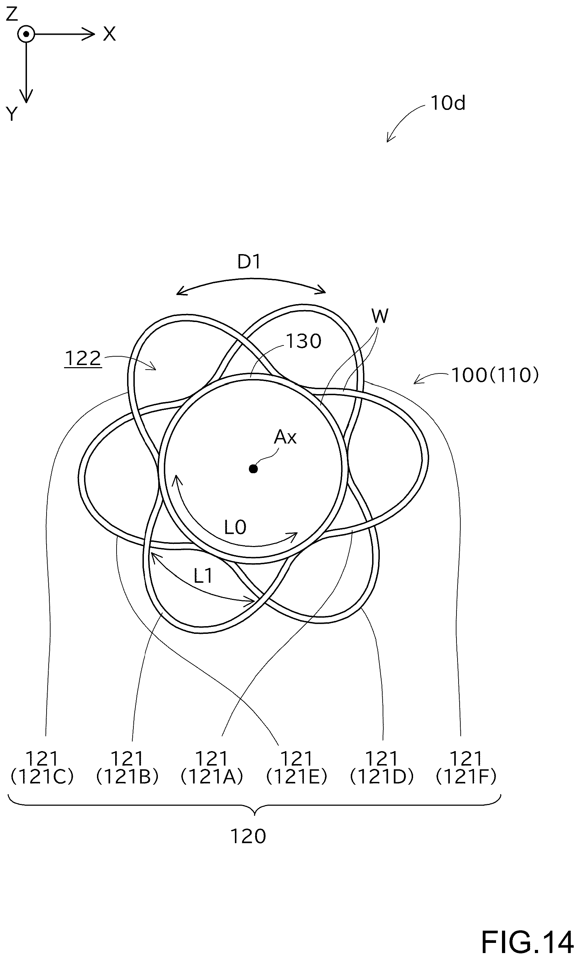

FIG. 13 is an explanatory view illustrating an external appearance configuration of a stent 10d in a fifth embodiment, and FIG. 14 is an explanatory view illustrating a planar configuration of the stent 10d in the fifth embodiment. Hereinafter, in the configuration of the stent 10d of the fifth embodiment, the same components as those of the stent 10 of the first embodiment described above will be denoted by the same reference signs, and the description thereof will be omitted as appropriate.

The stent 10d of the fifth embodiment is different from the stent 10 of the first embodiment in the configuration of the locking portion 120. In particular, in the stent 10d of the fifth embodiment, the locking portion 120 has six linear bodies 121 (121A, 121B, 121C, 121D, 121E, and 121F). Similarly to the stent 10 of the first embodiment, the three linear bodies 121A, 121B, and 121C among the six linear bodies 121 are arranged side by side continuously in the circumferential direction D1 of the tubular portion 110. The remaining three linear bodies 121D, 121E, and 121F are arranged side by side continuously in the circumferential direction D1 of the tubular portion 110 at the same positions as those of the three linear bodies 121A, 121B, and 121C in the direction of the center axis Ax (longitudinal direction) of the tubular portion 110. However, as illustrated in FIG. 14, when viewed in the direction of the center axis Ax of the tubular portion 110, the three linear bodies 121A, 121B, and 121C and the remaining three linear bodies 121D, 121E, and 121F have a relationship of being shifted by a predetermined angle (for example, 60 degrees) along the circumferential direction D1. Thus, when viewed in the direction of the center axis Ax of the tubular portion 110, one of the three linear bodies 121A, 121B, and 121C and one of the remaining three linear bodies 121D, 121E, and 121F are arranged so as to partially overlap each other. In the present embodiment, the six linear bodies 121 have substantially the same shape and size. One of the three linear bodies 121A, 121B, and 121C is an example of a first linear body, and one of the remaining three linear bodies 121D, 121E, and 121F is an example of a third linear body. One of the holes 11 formed in the entwined portions 101 through which the three linear bodies 121D, 121E, and 121F pass is an example of a third hole formed in a third entwined portion.

The stent 10d having such a configuration can be manufactured by performing the operation of extending the wire W to be hooked on the pins PN in a zigzag manner so that three large V-shaped portions convex downward are formed, and then, performing again the same operation from a position shifted by a predetermined angle (for example, 60 degrees), in the forming step of the linear bodies 121 of the locking portion 120 in the manufacturing method of the stent 10 of the first embodiment described above (the step illustrated in the column D of FIG. 5).

In the stent 10d of the fifth embodiment, similarly to the stent 10 of the first embodiment, the locking portion 120 has the first linear body 121 that passes through the first hole 11 formed in the first entwined portion 101 that is one of the plurality of entwined portions 101. Thus, the movement of a portion of the first linear body 121 passing through the first hole 11 is restricted, thereby suppressing the movement of the linear body 121, and a decrease in the anchor function of the locking portion 120 due to the movement can be suppressed.

In the stent 10d of the fifth embodiment, the locking portion 120 has another linear body (hereinafter, also referred to as a "third linear body") 121 arranged side by side with the first linear body 121 in the circumferential direction D1 of the tubular portion 110. The third linear body 121 passes through the hole (hereinafter also referred to as a "third hole") 11 formed in one entwined portion (hereinafter also referred to as a "third entwined portion") 101 among the plurality of entwined portions 101. When viewed in the direction of the center axis Ax of the tubular portion 110, the first linear body 121 and the third linear body 121 are arranged so as to partially overlap each other. Thus, according to the stent 10d of the fifth embodiment, a portion of the locking portion 120 in contact with the body wall has a planar spread, the stress concentration on the body wall by the locking portion 120 can be effectively alleviated, and the occurrence of bleeding or perforation in the body wall can be effectively suppressed.

### F. Sixth Embodiment:

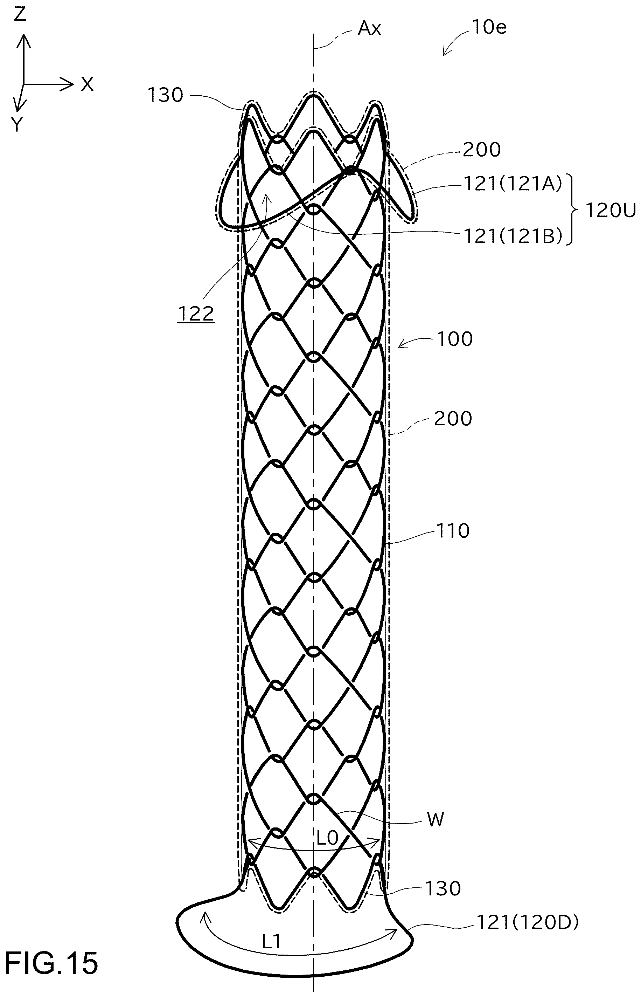

FIG. 15 is an explanatory view illustrating an external appearance configuration of a stent 10e in a sixth embodiment. Hereinafter, in the configuration of the stent 10e of the sixth embodiment, the same components as those of the stent 10 of the first embodiment described above will be denoted by the same reference signs, and the description thereof will be omitted as appropriate.

In addition to the locking portion 120 (hereinafter, for convenience, referred to as an "upper locking portion 120U") having the same configuration as that of the stent 10 of the first embodiment, the stent 10e of the sixth embodiment includes another locking portion 120 (hereinafter, for convenience, referred to as a "lower locking portion 120D") arranged at a different position (in particular, a lower position) from the position of the upper locking portion 120U in the direction of the center axis Ax of the tubular portion 110. Similarly to the upper locking portion 120U, the lower locking portion 120D protrudes to the outside of the tubular portion 110 when the tubular portion 110 is expanded.

The configuration of the lower locking portion 120D is the same as the configuration of the upper locking portion 120U except for the shape of each linear body 121 described below. That is, the lower locking portion 120D has three linear bodies 121, and each linear body 121 is formed so as to pass through the hole 11 formed in the entwined portion 101. The lower locking portion 120D is an example of a second locking portion, the linear body 121 constituting the lower locking portion 120D is an example of a fourth linear body, and the hole 11 formed in the entwined portion 101 through which the linear body 121 constituting the lower locking portion 120D passes is an example of a fourth hole formed in a fourth entwined portion.

When viewed in the direction of the center axis Ax of the tubular portion 110, the entire shape of each linear body 121 constituting the lower locking portion 120D is a substantially fan shape. That is, the entire shape of each linear body 121 constituting the lower locking portion 120D is a shape in which the width becomes wider as a distance from the connection portion with the tubular portion 110 is increased. Thus, a width L0 of the connection portion with the tubular portion 110 in each linear body 121 is smaller than a width L1 of another portion.

The stent 10e having such a configuration can be manufactured by performing the forming step of the linear bodies 121 of the locking portion 120 in the manufacturing method of the stent 10 of the first embodiment described above (the step illustrated in the column D of FIG. 5) while changing the shape of the linear body 121 at two positions of the position where the upper locking portion 120U is formed and the position where the lower locking portion 120D is formed.

In the stent 10e of the sixth embodiment, similarly to the stent 10 of the first embodiment, the locking portion 120 (the upper locking portion 120U) has the first linear body 121 that passes through the first hole 11 formed in the first entwined portion 101 that is one of the plurality of entwined portions 101. Thus, the movement of a portion of the first linear body 121 passing through the first hole 11 is restricted, thereby suppressing the movement of the linear body 121, and a decrease in the anchor function of the upper locking portion 120U due to the movement can be suppressed.

In addition to the upper locking portion 120U, the stent 10e of the sixth embodiment includes the lower locking portion 120D that protrudes to the outside of the tubular portion 110 when the tubular portion 110 is expanded. The lower locking portion 120D is arranged at a different position from the position of the upper locking portion 120U in the direction of the center axis Ax of the tubular portion 110. The lower locking portion 120D has the linear body (hereinafter also referred to as a "fourth linear body") 121 that passes through the hole (hereinafter also referred to as a "fourth hole") 11 formed in one entwined portion (hereinafter also referred to as a "fourth entwined portion") 101 among the plurality of entwined portions 101. The fourth linear body 121 has a shape in which the width of the connection portion with the tubular portion 110 is smaller than the width of another portion. Thus, according to the stent 10e of the sixth embodiment, a wide range portion of the linear body 121 constituting the lower locking portion 120D comes into contact with the body wall, the stress concentration on the body wall by the linear body 121 can be effectively alleviated, and the occurrence of bleeding or perforation in the body wall can be effectively suppressed. When the end portion of the stent 10e is pulled in the case where the stent 10e is occluded again, since the width of the connection portion of the linear body 121 constituting the lower locking portion 120D is small, the linear body 121 is easily reversed, and as a result, the facilitation of the removal of the stent 10e can be achieved.

### G. Another Embodiment:

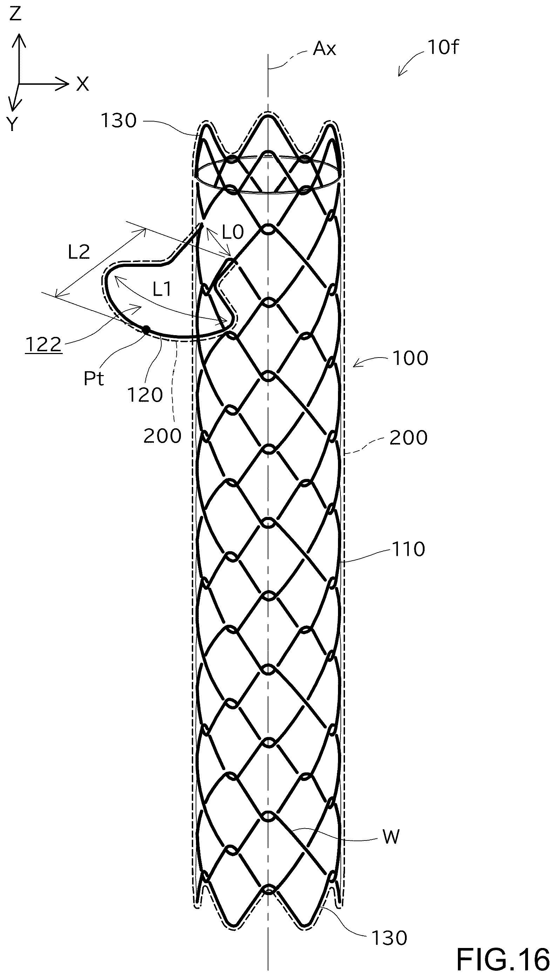

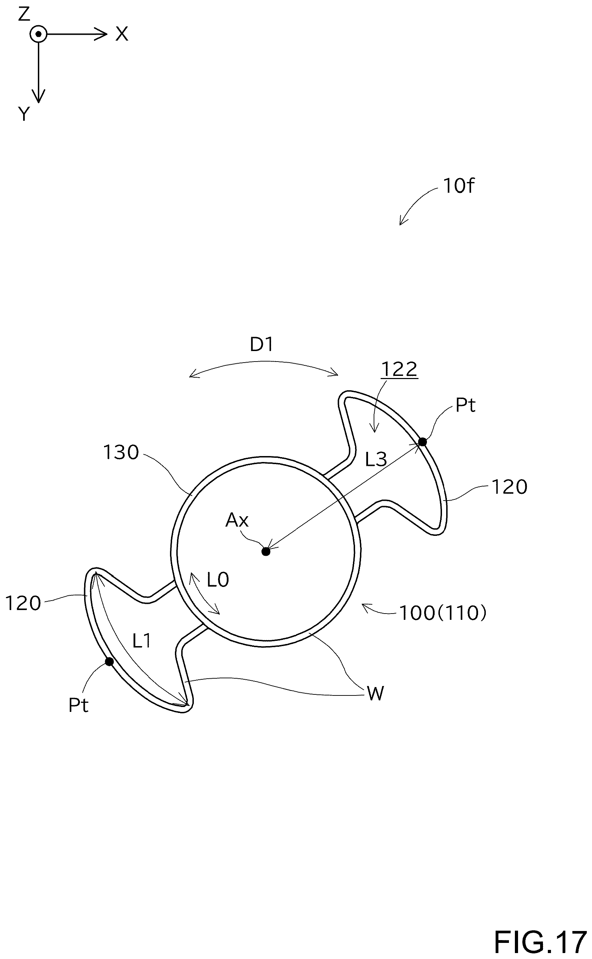

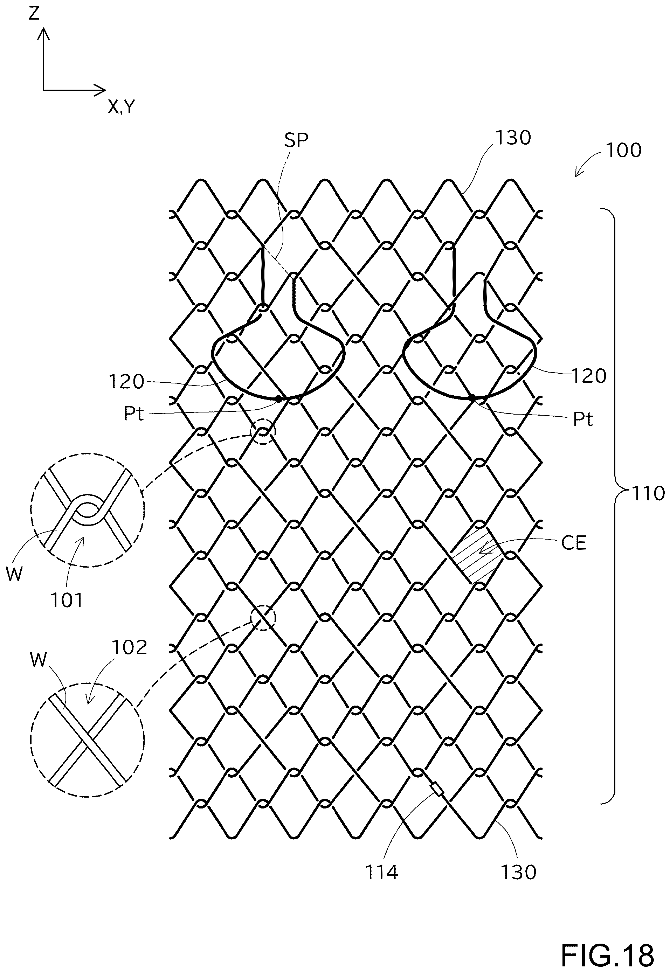

FIG. 16 is a perspective view illustrating an external appearance configuration of a stent 10f in another embodiment, and FIG. 17 is an explanatory view illustrating a planar configuration of the stent 10f in the another embodiment. FIG. 18 is a development view in which the main body part 100 in the stent 10f of the another embodiment is developed in the circumferential direction D1. Hereinafter, in the configuration of the stent 10f of the another embodiment, the same components as those of the stent 10 of the first embodiment described above will be denoted by the same reference signs, and the description thereof will be omitted as appropriate.

In the stent 10f of the another embodiment, two locking portions 120 are arranged side by side discretely and at equal intervals in the circumferential direction D1 in the vicinity of the upper end of the tubular portion 110 (at a position slightly lower than the upper end). In the present embodiment, each linear body 121 in each of the above-described embodiments is referred to as a locking portion 120. The locking portion 120 is formed by a part of the wire W constituting the entwined portion 101. More specifically, as illustrated in FIG. 18, the locking portion 120 is a substantially fan-shaped portion formed by providing a large curved slack to a part of one portion of the wire W constituting the entwined portion 101 (a substantially linear portion in another entwined portion 101, such as a specific portion SP virtually shown by a dash-dotted line in FIG. 18).

As illustrated in FIGS. 16 and 17, when viewed in the direction of the center axis Ax of the tubular portion 110, the locking portion 120 has a substantially fan shape as a whole. That is, the locking portion 120 has a shape in which the width becomes wider as a distance from the connection portion with the tubular portion 110 is increased. Thus, a width L0 of the connection portion with the tubular portion 110 in the locking portion 120 is smaller than a width L1 of another portion. In the present embodiment, the maximum width L1 of the locking portion 120 is larger than a protruding length L2 of the locking portion 120, but the present embodiment is not limited thereto. For example, the relationship of L1 ≥ 2/3 × L2 may be satisfied. In the present embodiment, the locking portion 120 is bent in a curved shape over the entire length, and does not have a folded portion. The cover 200 is arranged in the entire area 122 surrounded by the wire W constituting the locking portion 120.

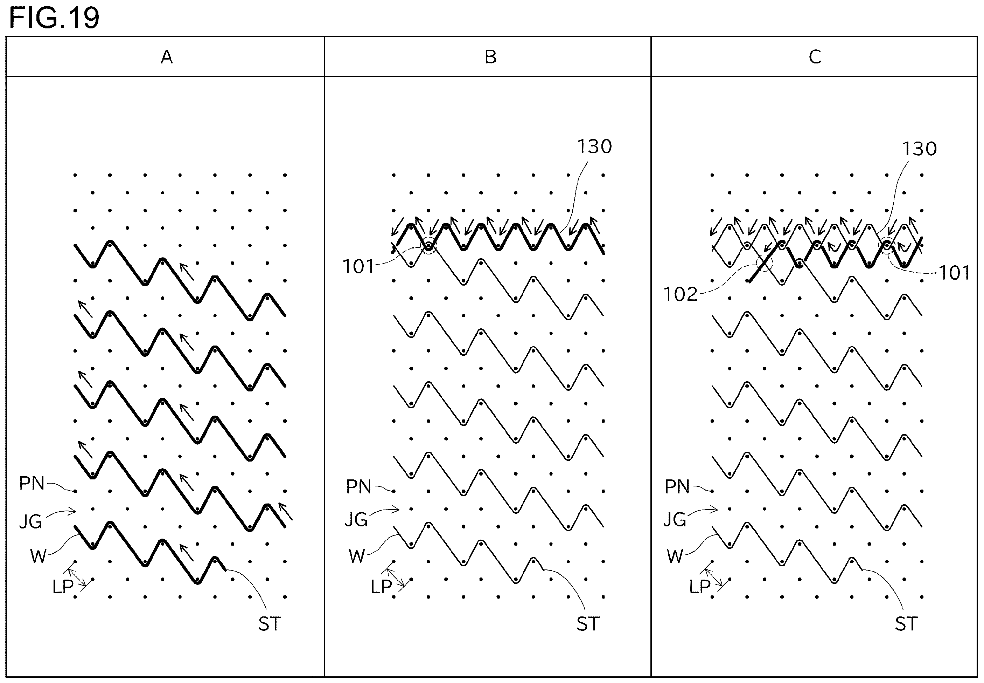

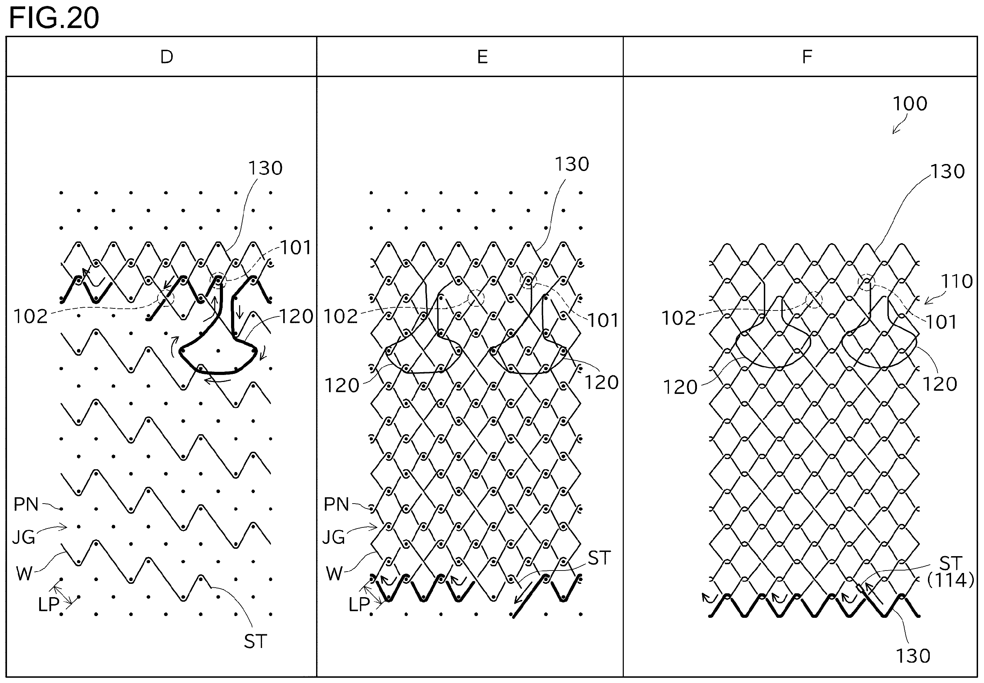

FIGS. 19 and 20 are explanatory views illustrating a weaving method of the main body part 100 in the stent 10f of the another embodiment. First, as illustrated in the column A of FIG. 19, an end portion of the wire W is fixed to the pin PN at a predetermined position of the jig JG as a start point ST, and the wire W is extended and hooked on the pins PN in a zigzag manner from the start point ST (similar to the step illustrated in the column A of FIG. 4 described above). Next, as illustrated in the column B of FIG. 19, following the formation state of the wire W in the column A of FIG. 19, the wire W is extended and hooked on the pins PN in a zigzag manner from a position corresponding to the upper end of the tubular portion 110 so that six V-shaped portions (the protruding portions 130) convex upward are formed (similar to the step illustrated in the column B of FIG. 4 described above).

Next, as illustrated in the column C of FIG. 19, following the formation state of the wire W in the column B of FIG. 19, the wire W is extended and hooked on the pins PN in a zigzag manner so that four V-shaped portions convex downward are formed. More specifically, an operation of extending the wire W obliquely downward and leftward by 1 × LP to be hooked on the lower side of the pin PN and an operation of extending the wire W obliquely upward and leftward by 1 × LP to be hooked on the upper side of the pin PN are repeated four times. Thereafter, the wire W is extended obliquely downward and leftward by 2×LP.

Next, as illustrated in the column D of FIG. 20, following the formation state of the wire W in the column C of FIG. 19, the same operation as that in the column C of FIG. 19 is repeated until the wire W reaches a position corresponding to the lower end of the tubular portion 110. However, at a position where the locking portion 120 is formed, the locking portion 120 before processing is formed. More specifically, at the position where the locking portion 120 is formed, an operation of providing a slack to a part of the wire W constituting the entwined portion 101 and hooking the wire W on the pins PN so as to have a substantially fan shape is performed. The column E of FIG. 20 illustrates a state in which the wire W has reached the position corresponding to the lower end of the tubular portion 110 after forming the two locking portions 120 before processing.

Next, as illustrated in the column F of FIG. 20, following the formation state of the wire W in the column E of FIG. 20, the wire W is extended and hooked on the pins PN in a zigzag manner so that six V-shaped portions (the protruding portions 130) convex downward are formed. That is, the protruding portions 130 are formed by an operation of extending the wire W obliquely downward and leftward by 1 × LP to be hooked on the lower side of the pin PN and an operation of extending the wire W obliquely upward and leftward by 1 × LP to be hooked on the upper side of the pin PN. After the sixth protruding portion 130 is formed, the wire W is extended to the start point ST, and both ends of the wire W are connected at the start point ST by, for example, forming a caulking portion 114 by caulking. By the above-described steps, the main body part 100 before processing of the locking portion 120 is manufactured.

Next, for example, by using a disc-shaped mold, the locking portion 120 before processing is fixed in a state of being lifted so as to protrude from the tubular portion 110 to the outer peripheral side, and heat treatment for shape memory (for example, at 350 to 600°C for 5 to 30 minutes) is performed. Accordingly, the locking portion 120 is formed so as to protrude from the tubular portion 110 to the outer peripheral side during expansion.

Finally, the cover 200 is formed at a predetermined position of the main body part 100. By the above-described steps, the manufacturing of the stent 10f of the present embodiment is completed.

### H. Modifications:

The technique disclosed in the present specification is not limited to the embodiments described above, and various modifications can be made without departing from the gist thereof. For example, the following modifications can be made.

The configuration of the stent 10 in the above-described embodiment is solely an example, and various modifications can be made. For example, the shape of the linear body 121 constituting the locking portion 120 is not limited to a bowl shape, a trapezoidal shape, a fan shape, or the like, and any shape can be adopted. The linear body 121 constituting the locking portion 120 may pass through the hole 11 of the entwined portion 101 at only one position. In a case where the linear body 121 constituting the locking portion 120 passes through the first hole 11 of the first entwined portion 101 and the second hole 11 of the second entwined portion 101, the first entwined portion 101 and the second entwined portion 101 may be directly adjacent to each other in the circumferential direction D1 without interposing another entwined portion 101 therebetween. In a case where the locking portion 120 has a plurality of linear bodies 121, at least one of the plurality of linear bodies 121 may pass through the hole 11 of the entwined portion 101. The linear body 121 constituting the locking portion 120 may not be positioned in the same virtual plane. The forming positions and the number of the locking portions 120 (the linear bodies 121) in the tubular portion 110 can be freely changed. The main body part 100 may not have the protruding portion 130.

The manufacturing method of the stent 10 in the above-described embodiment is solely an example, and various modifications can be made. For example, in the above-described embodiment, when the main body part 100 is manufactured by hook weaving, weaving is performed so that the six V-shaped portions are arranged in the circumferential direction D1. However, the number of the V-shaped portions arranged in the circumferential direction D1 may be equal to or less than five, or may be equal to or more than seven. The technique disclosed in the present specification can be similarly applied to a stent manufactured by another weaving method (for example, cross weaving) without limiting to a stent manufactured by hook weaving. The weaving method of the main body part 100 does not need to be hook weaving as a whole, and may partially include hook weaving.

In the above-described embodiment, the stent 10 is a covered stent that has the cover 200. However, the technique disclosed in the present specification can be similarly applied to an uncovered stent that does not have the cover 200.

### DESCRIPTION OF REFERENCE NUMERALS

10: stent
11: hole
20: living body lumen
21: body wall
100: main body part
101: entwined portion
102: intersecting portion
110: tubular portion
114: caulking portion
120: locking portion
120D: lower locking portion
120U: upper locking portion
121: linear body
122: area
130: protruding portion
200: cover
Ax: center axis
CE: cell
D1: circumferential direction
JG: jig
P1: locking portion formation start point
P2: reference point
PN: pin
Pt: outermost peripheral point
SP: specific portion
ST: start point
W: wire

## Claims

1. A stent comprising:
an expandable tubular portion that is formed by weaving a wire and has a plurality of entwined portions at each of which the wire intersects with itself in a hook shape and is entwined with itself; and
a locking portion that protrudes to an outside of the tubular portion when the tubular portion is expanded, wherein
the locking portion has a first linear body that passes through a first hole formed in a first entwined portion that is one of the plurality of entwined portions.

2. The stent according to claim 1, wherein
the first linear body is constituted by a part of the wire.

3. The stent according to claim 1 or 2, wherein
the first linear body is positioned in the same virtual plane.

4. The stent according to any one of claims 1 to 3, wherein
the first linear body passes through a second hole formed in a second entwined portion that is one of the plurality of entwined portions and arranged side by side with the first entwined portion in a circumferential direction of the tubular portion.

5. The stent according to claim 4, wherein
another entwined portion, being one of the plurality of entwined portions, is positioned between the first entwined portion and the second entwined portion in the circumferential direction of the tubular portion.

6. The stent according to claim 4 or 5, wherein
the locking portion further has a second linear body that passes through the first hole and the second hole.

7. The stent according to claim 6, wherein
the second linear body is constituted by a part of the wire.

8. The stent according to claim 6 or 7, wherein
a length of the first linear body and a length of the second linear body are different from each other.

9. The stent according to any one of claims 1 to 8, wherein
the locking portion further has a third linear body arranged side by side with the first linear body in the circumferential direction of the tubular portion and passing through a third hole formed in a third entwined portion that is one of the plurality of entwined portions, and,
when viewed in a direction of a center axis of the tubular portion, the first linear body and the third linear body are arranged so as to partially overlap each other.

10. The stent according to any one of claims 1 to 9, further comprising:
a resin cover that covers at least a part of the tubular portion, wherein
the cover is arranged in an area surrounded by the first linear body constituting the locking portion.

11. The stent according to any one of claims 1 to 10, wherein
the first linear body has a shape in which a width of a connection portion with the tubular portion is larger than a width of another portion.

12. The stent according to claim 11, further comprising:
a second locking portion that is arranged at a different position from a position of the locking portion in the direction of the center axis of the tubular portion and protrudes to the outside of the tubular portion when the tubular portion is expanded, wherein
the second locking portion has a fourth linear body that passes through a fourth hole formed in a fourth entwined portion that is one of the plurality of entwined portions, and
the fourth linear body has a shape in which a width of a connection portion with the tubular portion is smaller than a width of another portion.
